Europäisches Patentamt

European Patent Office

Office européen des brevets

(19)

(11) Veröffentlichungsnummer: **0 424 535 A1**

# (12) EUROPÄISCHE PATENTANMELDUNG
## veröffentlicht nach Art. 158 Abs. 3 EPÜ

(21) Anmeldenummer: 89909912.1

(22) Anmeldetag: 21.04.89

(86) Internationale Anmeldenummer: PCT/SU89/00108

(87) Internationale Veröffentlichungsnummer: WO 90/12567 (01.11.90 90/25)

(51) Int. Cl.⁵: **A61K 9/52**

(43) Veröffentlichungstag der Anmeldung: **02.05.91 Patentblatt 91/18**

(84) Benannte Vertragsstaaten: **AT DE FR GB IT NL**

(71) Anmelder: **MOSKOVSKY AVTOMOBILESTROITELNY INSTITUT (VTUZ-ZIL)**
ul. Avtozavodskaya, 16
Moscow, 109268(SU)

Anmelder: **VSESOJUZNY NAUCHNO-ISSLEDOVATELSKY INSTITUT GELMINTOLOGII IMENI AKADEMIKA K.I. SKRYABINA**
ul. B.Cheremushkinskaya, 28
Moscow, 117259(SU)

Anmelder: **VSESOJUZNY NAUCHNO-ISSLEDOVATELSKY INSTITUT MEDITSINSKIKH POLIMEROV**
Nauchny proezd, 10
Moscow, 117246(SU)

(72) Erfinder: **GRIGORIANTS, Igor Konstantinovich**
Leninsky pr., 69-135
Moscow, 117296(SU)
Erfinder: **BALABUSHEVICH, Alexandr Georgievich**
ul. Isakovskogo, 26-2
Moscow, 123631(SU)
Erfinder: **TRIKHANOVA, Galina Andreevna**
ul. Rokotova, 7-2-86
Moscow, 117593(SU)
Erfinder: **ARBUZOVA, Larisa Alexandrovna**
Orekhovy bulvar, 39-2
Moscow, 115573(SU)
Erfinder: **LIPANOV, Alexei Matveevich**
ul. B.Gruzinskaya, 39-122
Moscow, 123056(SU)
Erfinder: **STUKALOVA, Nadezhda Pavlovna**
ul. Turistskaya, 25-1
Moscow, 123514(SU)
Erfinder: **DEMIDOV, Nikolai Vasilievich**
Sevastopolsky pr., 14-188
Moscow, 113447(SU)
Erfinder: **TIKHOMIROVA, Nina Nikolaevna**
ul. Baikalskaya, 34-95
Moscow, 107207(SU)
Erfinder: **BOCHAROV, Mikhail Yakovlevich**
ul. Sudostroitelnaya, 28-7
Moscow, 115407(SU)
Erfinder: **LYAKHNOVICH, Sergei Vladimirovich**
Lomonosovsky pr., 18-132
Moscow, 217296(SU)

(74) Vertreter: **Nix, Frank Arnold, Dr. et al**
Kröckelbergstrasse 15
W-6200 Wiesbaden(DE)

(54) **VORRICHTUNG ZUR KONTROLLIERBAREN DOSIERUNG VON WIRKSTOFFEN IN EIN FUNKTIONIERUNGSMEDIUM.**

(57) Die Vorrichtung zur kontrollierbaren Dosierung von Wirkstoffen in ein Funktionierungsmedium ent-

hält ein zylinderförmiges Gehäuse (1) mit mindestens einem Mittel zur Verbindung des Gehäuses (1) mit dem Funktionierungsmedium und im Innern des Gehäuses (1) hintereinander angeordnete Abteilungen (6), die mit Wirkstoff (7) gefüllt und durch Trennwände (8) getrennt sind. Zumindest an einem Stirnende des Gehäuses (1) auf der Seite der Unterbringung des Funktionierungsmediums ist ein Netz (5) und ein Aufsatzstück (2) angebracht, das in Form einer Hülse mit einem in Richtung des Funktionierungsmediums konkaven Boden ausgebildet ist, bei dem zumindest ein Teil die Form eines Kegelstumpfes (3) aufweist. In der kleineren Grundfläche des Kegelstumpfes (3) ist eine Öffnung (4) ausgebildet, die das Mittel zur Verbindung des Gehäuses (1) mit dem Funktionierungsmediums darstellt. Jede Trennwand (8) ist in Form einer polymeren Matrize mit darin dispergiertem Wirkstoff (7) ausgebildet, wobei die Konzentration des Wirkstoffs (7) in der polymeren Matrize in den Grenzen von 1 bis 70% liegt .

FIG.1

FIG.2

# VORRICHTUNG ZUR KONTROLLIERBAREN DOSIERUNG VON WIRKSTOFFEN IN EIN FUNKTIONIERUNGS-MEDIUM

## Technisches Gebiet

Die Erfindung bezieht sich auf Vorrichtungen zum Einführen fester flüssiger und gasförmiger Medikamente und anderer Stoffe in einen Organismus, insbesondere auf eine Vorrichtung zur kontrollierbaren Dosierung von Wirkstoffen in ein Funktionierungsmedium.

## Zugrundeliegender Stand der Technik

Es ist ein Ausscheidungssystem der kontrollierbaren Zuführung bekannt (US, A, 42290153), das einen zylinderförmigen Behälter mit einem Arzneimittel enthält, dessen Wand aus einem porösen Gewebestoff besteht, dessen Poren mit Hydrogel ausgefüllt sind. Durch die Hydrogelschicht diffundiert die Flüssigkeit aus dem umgebenden Medium zum Arzneimittel; zurück in das Medium diffundiert das Arzneimittel in Form einer Lösung. Mit Hilfe dieses Systems ist es nicht möglich, das Arzneimittel für eine Zeitperiode von mehr als neunzig bis einhundertzwanzig Tagen zu dosieren. Auch ist es unmöglich, eine Dosierung von zwei unverträglichen Arzneimitteln zu gewährleisten.

Bekannt ist eine mit einem Netz bedeckte Pille (US, A, 4326522), die aus zylindrischen Kapseln besteht, welche aus einem biologisch abbaubaren Polymer, vermischt mit Arzneimittel, hergestellt sind. Die zylindrischen Kapseln sind untereinander durch biegsame federnde Elemente verbunden, die es gestatten, beim Gelangen der Pille in den Magen der Wiederkäuer ihr eine solche Form zu verleihen, die das Herausführen der Pille aus den Magen der Wiederkäuer verhindert. Die ganze Pille ist mit einem Netz bedeckt, das die polymere Matrize mit Wirkstoff, die unter der Wirkung der Magenflüssigkeit zerfällt, festhält. Die genannte Pille gewährleistet keine kontrollierbare Dosierung der Arzneimittel, sie ermöglicht es, nur einen Stoff im Laufe von höchstens dreissig Tagen zu dosieren.

Es ist eine Vorrichtung zur kontrollierbaren Dosierung von Wirkstoffen in ein Funktionierungsmedium bekannt (US, A, 4381780), die ein zylinderförmiges Gehäuse mit mindestens einem Mittel zur Verbindung des Gehäuses mit dem Funktionierungs-Medium und im Innern des Gehäuses hintereinander angeordnete Abteilungen enthält, die mit Wirkstoff gefüllt und durch Trennwände aus biologisch abbaubarem Stoff (Papier, Karton) getrennt sind, welche beim Kontakt mit der Magenflüssigkeit zerstört werden, was zur Ausscheidung des Wirkstoffs in das Funktionierungsmedium führt. Die Dosiergeschwindigkeit wird durch die Geschwindigkeit der Zerstörung der Trennwände bestimmt. Diese Vorrichtung gewährleistet keine beständige Dosiergeschwindigkeit des Wirkstoffs, d.h. sie gewährleistet keine Dosierung der Wirkstoffe im Laufe einer längeren Zeit mit einer vorgegebenen Konzentration. Dies ist darauf zurückzuführen, dass die Zerstörung der Trennwand zum Ausscheiden einer Einzeldosis von Wirkstoff führt, dessen Konzentration im Laufe eines Tages (24 Stunden) vom Maximalwert bis zum Minimalwert ständig abnimmt. Diese Vorrichtung erlaubt es auch nicht, mehrere Wirkstoffe gleichzeitig zu dosieren, sie gestattet es nicht, in der Anfangspediode des Funktionierens eine erhöhte Wirkstoffdosis auszuscheiden.

## Offenbarung der Erfindung

Der Erfindung liegt die Aufgabe zugrunde, eine Vorrichtung zur kontrollierbaren Dosierung von Wirkstoffen in ein Funktionierungsmedium mit solch einer konstruktiven Ausführung zu entwickeln, die es ermöglicht, durch einen kontrollierbaren Einlauf der Flüssigkeit aus dem Funktionierungsmedium ins Gehäuse und eine Erweiterung der Kontaktfront des Funktionierungsmediums mit dem Wirkstoff sowie dank dem Vorhandensein eines zusätzlichen Wirkstoffvolumens im Gehäuse eine Dosierung des Wirkstoffs im Laufe einer längeren Zeit mit der vorgegebenen Konzentration zu verwirklichen sowie gleichzeitig mehrere Wirkstoffe zu dosieren und in der Anfangsperiode der Funktionierung eine erhöhte Wirkstoffdosis auszuscheiden.

Die gestellte Aufgabe wird dadurch gelöst, dass in der Vorrichtung zur kontrollierbaren Dosierung von Wirkstoffen in das Funktionierungsmedium, die ein zylinderförmiges Gehäuse mit mindestens einem Mittel zur Verbindung des Gehäuses mit dem Funktionierungsmedium und im Innern des Gehäuses hintereinander angeordnete Abteilungen, die mit Wirkstoff gefüllt und durch Trennwände getrennt sind, enthält, erfindungsgemäss zumindest an einem Gehäusestirnende auf der Seite der Unterbringung des Funktionierungsmediums ein Netz und ein Aufsatzstück angebracht ist, das in Form einer Hülse mit einem in Richtung des Funktionierungsmediums konkaven Boden ausgebildet ist, von dem mindestens ein Teil die Form eines Kegelstumpfes hat, bei dem die kleinere Grundfläche eine Öffnung aufweist, die das Verbindungsmittel des Gehäuses mit dem Funktionierungsmedium darstellt, jede Trennwand in Form einer polymeren Matrize mit darin dispergiertem Wirkstoff ausgeführt ist, wobei die Konzentration des Wirkstoffes in der polymeren Matrize in den Grenzen von 1 bis 70% liegt.

Es ist zweckmässig, die Vorrichtung mit einem Einsatz zu versehen, der im Gehäuseinnern untergebracht und an den Gehäusewänden befestigt ist, wobei der Einsatz den Innenraum des Gehäuses in zwei Teile aufteilt, die voneinander isoliert sind, an jedem Gehäusestirnende ein Netz und ein Aufsatzstück angebracht ist, dessen Öffnung mit dem entsprechenden Teil des Gehäuseinnenraumes verbunden ist.

Es ist auch zweckmässig, die Vorrichtung mit einem Einsatz zu versehen, der im Gehäuseinnern untergebracht und an den Gehäusewänden befestigt ist, wobei der Einsatz den Gehäuseinnenraum mindestens in drei voneinander isolierte Teile aufteilt, und an jedem Gehäusestirnende ein Netz und ein Aufsatzstück angebracht ist, dessen Öffnung , die das Mittel zur Verbindung des Gehäuses mit dem Funktionierungsmedium darstellt, mit dem entsprechenden Teil des Gehäuseinnenraumes verbunden ist, in den anderen Teilen des Gehäuseinnerraumes ist das Mittel zur Verbindung des Gehäuses mit dem Funktionierungsmedium in Form einer Öffnung in der Gehäuseseitenwand ausgebildet.

Die erfindungsgemässe Vorrichtung zur kontrollierbaren Dosierung von Wirkstoffen in das Funktionierungsmedium gestattet es, die Wirkstoffe in das Funktionierungsmedium eine längere Zeit mit einer kontrollierbaren Geschwindigkeit zu dosieren. Bei Bedarf gestattet die Vorrichtung in der Anfangsperiode der Funktion, eine erhöhte Wirkstoffdosis auszuscheiden. Diese Vorrichtung ermöglicht es auch, gleichzeitig mehrere Wirkstoffe mit einer Dosierzeit derselben von hundertachtzig bis zweihundert Tage zu dosieren. Die erfindungsgemässe Vorrichtung, die man für die Heilung und Prophylaxe bei Tieren verwendet, gestattet es, die Gewichtszunahme eines jeden Tieres um sechs bis fünfundzwanzig Kilogramm zu erhöhen.

Kurze Beschreibung der Zeichnungen

Nachstehend soll die Erfindung an Hand von konkreten Ausführungsbeispielen und Zeichnungen näher erläutert werden; in diesen zeigt;
Fig. 1 die Gesamtansicht der Vorrichtung zur kontrollierbaren Dosierung von Wirkstoffen mit einem Aufsatzstück am Gehäusestirnende, Längsschnitt, gemäss der Erfindung;
Fig. 2 die Gesamtansicht der Vorrichtung zur kontrollierbaren Dosierung von Wirkstoffen mit zwei Aufsatzstücken und einem Einsatz, der den Gehäuseinnenraum in zwei Teile aufteilt, Längsschnitt, gemäss der Erfindung;
Fig. 3 die Gesamtansicht der Vorrichtung zur kontrollierbaren Dosierung von Wirkstoffen mit einem Einsatz , der den Gehäuseinnenraum in vier Teile aufteilt, Längsschnitt, gemäss der Erfindung;
Fig. 4 die Vorrichtung zur kontrollierbaren Dosierung von Wirkstoffen, Querschnitt entlang der Linie IV-IV von Fig. 3;
Fig. 5 die Ansicht in Pfeilrichtung A von Fig. 3 mit einem Querschnitt durch das Gehäuse und das Aufsatzstück.

Beste Ausführungsform der Erfindung

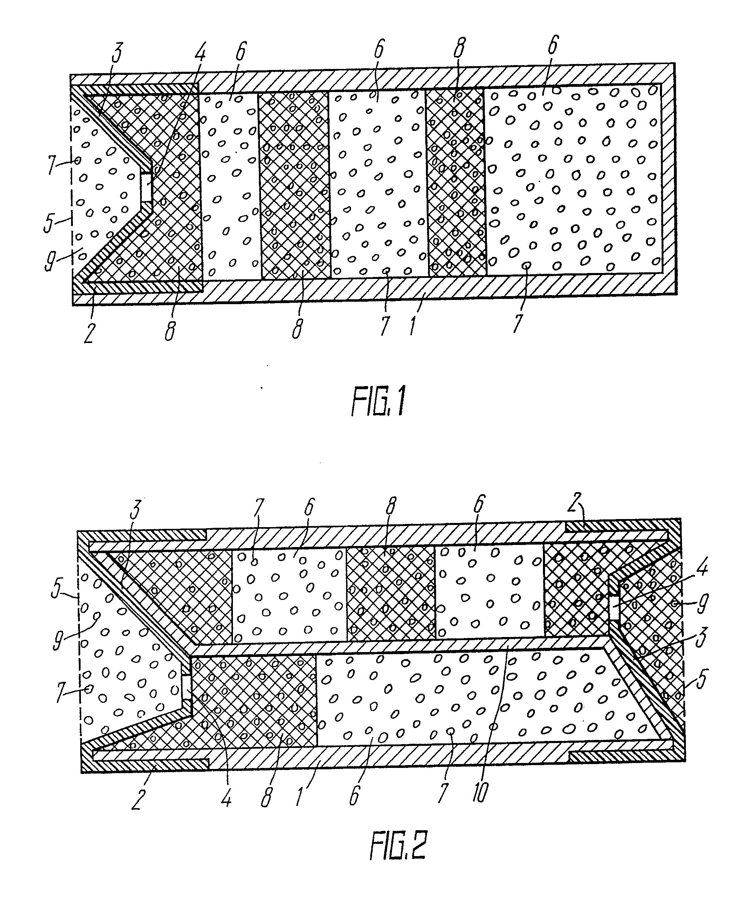

Die Vorrichtung zur kontrollierbaren Dosierung von Wirkstoffen in das Funktionierungsmedium enthält ein in Form eines Hohlzylinders aus Metall oder einemanderen schweren und biologisch unschädlichen Material ausgeführtes Gehäuse 1 (Fig. 1). Mindestens an einem Stirnende des Gehäuses 1 ist auf der Unterbringungsseite des Funktionierungsmediums ein Aufsatzstück 2 (in der Fig. ist das Funktionierungsmedium nicht dargestellt) angebracht. Im vorliegenden Beispiel ist das Aufsatzstück 2 an nur einem Stirnende des Gehäuses 1 angebracht. Das Aufsatzstück 2 ist in Form einer Hülse mit einem in Richtung des Funktionierungsmediums konkaven Boden ausgebildet, der die Form eines Kegelstumpfes 3 aufweist. Die kleinere Grundfläche des Kegelstumpfes 3 ist mit einer Öffnung 4 versehen, die als Verbindungsmittel des Gehäuses 1 mit dem Funktionierungsmedium dient. Auch mindestens an einem Stirnende des Gehäuses 1 auf der Seite der Unterbringung des Funktionierungsmediums ist ein Netz 5 angebracht. Im vorliegenden Falle ist das Netz 5 an nur einem Stirnende des Gehäuses 1 angebracht. Im Innern des Gehäuses 1 sind hintereinander drei Abteilungen 6 mit Wirkstoff 7 untergebracht, die durch drei Trennwände 8 getrennt sind, welche in Form einer polymeren Matrize mit darin dispergiertem Wirkstoff 7 ausgeführt sind. Ein Teil 9 des Aufsatzstücks 2, der durch das Netz 5 und den Boden des Aufsatzstücks 2 begrenzt ist, ist mit Wirkstoff 7 gefüllt. In den Abteilungen 6, in den Trennwänden 8 und im Teil 9 des Aufsatzstücks 2 werden verschiedene Wirkstoffe 7 verwendet. So kann z.B. der Teil 9 des Aufsatzstücks 2 mit einem Antiseptikum - Zinksulfat, die Abteilungen 6 mit einem Antibiotikum - Vitatetrin, die Trennwände 8 - mit Glukose gefüllt sein. In einer jeden Trennwand 8 kann die Konzentration des Wirkstoffes 7 in der polymeren Matrize im Bereich von 1 bis 70% liegen.Die Einführung von weniger als 1% Wirkstoff 7 in die polymere Matrize führt zu einem hohen Widerstand der polymeren Matrize gegen den Flüssigkeitsstrom aus dem Funktionierungsmedium und als Folge davon zu einer unbedeutenden Ausscheidungsgeschwindigkeit des Wirkstoffes 7. Die Einführung von mehr als 70% Wirkstoff 7 in die polymere Matrize führt zu der Auflockerung der polymeren Matrize, was zu einer unkonttrollierbaren Ausscheidung des Wirkstoffes 7 führt.

Die erfindungsgemässe Vorrichtung zur kontrollierbaren Dosierung von wirkstoffen kann auch einen im Innern des Gehäuses 1 untergebrachten und an dessen Wänden befestigten Einsatz 10 (Fig. 2) haben. Der Einsatz 10 teilt den Innenraum des Gehäuses 1 in zwei voneinander isolierte Teile auf. Im vorliegenden Beispiel ist an jedem Stirnende des Gehäuses 1 ein Netz 5 und ein Aufsatzstück 2 angebracht. Die Öffnung 4 eines jeden Aufsatzstücks 5 ist mit dem entsprechenden Teil des Innenraums des Gehäuses 1 verbunden. Der Boden eines jeden Aufsatzstücks 2 hat die Form eines asymmetrischen Kegelstumpfes 3.

Verschiedene Kombinationen der Anordnung der Abteilungen 6 mit Wirkstoff 7 und der Trennwände 8 gewährleisten eine kontrollierbare Ausscheidungsgeschwindigkeit der Wirkstoffe 7. Die in Rede stehende Konstruktion der Vorrichtung gewährleistet eine gleichzeitige Dosierung von zwei nichtmischbaren Wirkstoffen 7. In einem Teil des Innenraums des Gehäuses 1 gibt es zwei Abteilungen 6 mit Wirkstoff und drei Trennwände 8. In dem anderen Teil des Innenraums des Gehäuses 1 gibt es eine Abteilung 6 und eine Trennwand 8. An einem Stirnende des Gehäuses 1 ist ein Teil 9 des Aufsatzstücks 2 mit Wirkstoff 7 und an dem anderen Stirnende des Gehäuses 1 ein Teil 9 des Aufsatzstücks 2 mit einer Komposition aus Polymer und Wirkstoff 7 gefüllt, wobei die Konzentration des Wirkstoffs 7 in der polymeren Matrize gleich 50% ist.

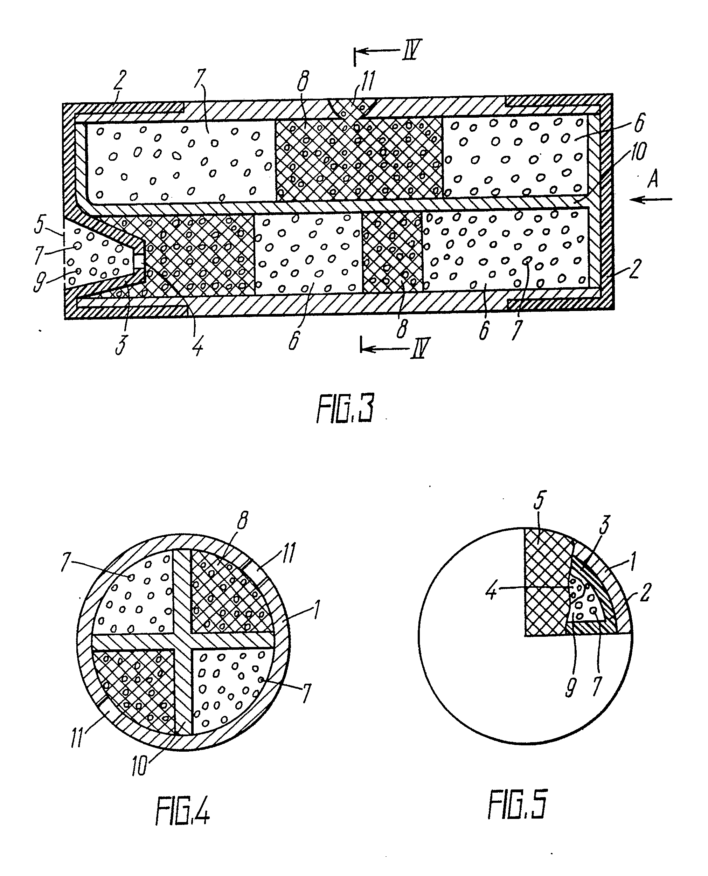

In Fig. 3 ist eine Vorrichtung zur kontrollierbaren Dosierung von Wirkstoffen mit einem Einsatz 10 dargestellt, der im Innern des Gehäuses 1 untergebracht und an dessen Wänden befestigt ist und den Innenraum des Gehäuses 1 mindestens in drei Teile aufteilt, die voneinander isoliert sind. Im vorliegenden Beispiel ist der Innenraum des Gehäuses 1 in vier isolierte Teile aufgeteilt. An jedem Stirnende des Gehäuses 1 ist ein Netz 5 und ein Aufsatzstück 2 angebracht. Die Öffnung 4 eines jeden Aufsatzstücks 2, die als Verbindungsmittel des Gehäuses 1 mit dem Funktionierungsmedium dient, ist mit einem entsprechenden Teil des Innenraums des Gehäuses 1 verbunden. In jedem der zwei anderen Teile des Innenraums des Gehäuses 1 ist das Mittel zur Verbindung des Gehäuses 1 mit dem Funktionierungsmedium in Form einer entsprechenden Öffnung 11 in der Seitenwand des Gehäuses 1 ausgebildet. In einem der Teile des Innenraums des Gehäuses 1, der mit dem Funktionierungsmedium über die Öffnung 4 des Aufsatzstücks 2 verbunden ist, sind zwei Abteilungen 6 und zwei Trennwände 8 vorhanden. In einem anderen der Teile des Innenraums des Gehäuses 1, der mit dem Funktionierungsmedium über die Öffnung 11 verbunden ist, sind zwei Abteilungen 6 und eine Trennwand 8 vorgesehen, wobei die Konzentration des Wirkstoffs 7 gleich 1% ist. Der Teil 9 des Aufsatzstücks 2 ist mit Wirkstoff 7 gefüllt. In einem weiteren Teil des Innenraums des Gehäuses 1 ist die Konzentration des Wirkstoffs in den Trennwänden 8 gleich 70%.

Fig. 4 zeigt den Querschnitt nach Linie IV-IV der Fig. 3 der Vorrichtung zur kontrollierbaren Dosierung von Wirkstoffen. In der Seitenwand des Gehäuses 1 sind zwei Öffnungen 11 ausgebildet. In Fig. 5 ist die Ansicht in Richtung des Pfeils A von Fig. 3 mit einem Querschnitt durch das Gehäuse 1 und das Aufsatzstück 2 dargestellt. Der Teil 9 des zweiten Aufsatzstücks 2 ist auch mit Wirkstoff 7 gefüllt.

Die Vorrichtung zur kontrollierbaren Dosierung von Wirkstoffen in das Funktionierungsmedium arbeitet in folgender Weise.

Bevor man die Vorrichtung in das Funktionierungsmedium, vorliegend in den Magen eines Wiederkäuers, einbringt, ist die Vorrichtung einsatzbereit zu machen. Zu diesem Zweck bringt man die Abteilung 6, die mit dem Wirkstoff 7 - dem Antihelminthenpräparat Tividin - in Form einer Schmelze bzw. einer zusammengepressten Masse gefüllt ist, im Inneren des Gehäuses 1 (Fig. 1) unter. Danach füllt man die Trennwand 8 ein, die aus einer Komposition besteht, die 30% Polyvinylchlorid-Plastisol und 70% Wirkstoff 7 - Tividin - enthält, der in der polymeren Matrize gleichmässig verteilt ist. Danach füllt man von neuen die Abteilung 6 mit Wirkstoff 7 - Tividin, den man von der nächsten Abteilung 6 durch eine Trennwand 8 aus einer Komposition, die 30% Polyvinylchlorid-Plastisol und 70% Wirkstoff 7 - Tividin - enthält, trennt. Die nächste Abteilung 6 füllt man wieder mit Wirkstoff 7 - Tividin. Dann setzt man in das Gehäuse 1 das Aufsatzstück 2 ein, das auf der dem Inneren des Gehäuses 1 zugekehrten Seite mit einer Komposition aus 40% Polyvinylchlorid-Plastisol und 60% Wirkstoff 7 - Tividin - gefüllt ist. Auf der dem Funktionierungsmedium zugekehrten Seite ist der Teil 9 des Aufsatzstücks 2 mit Wirkstoff 7 - Tividin -gefüllt. Nach dem Zusammenbau gelangt die Vorrichtung,wenn nötig, zur Wärmebehandlung zwecks Polymerhärtung.

Ist die Vorrichtung mit einem Einsatz 10 (Fig. 2) versehen, so bringt man in das Gehäuse 1 zunächst den Einsatz 10 ein, welcher den Innenraum des Gehäuses 1 in zwei isolierte Teile aufteilt. Man füllt jeden Teil mit verschiedenen Wirkstoffen 7 oder deren Gemischen. Die Trennwände 8, die Abteilungen 6 und der Teil 9 des Aufsatzstücks 2 des einen Teils des Gehäuses 1 enthalten Tividin als Wirkstoff 7. Der andere Teil des Gehäuses 1 enthält in den Abteilungen 6 Antiseptikum als Wirkstoff 7, das auch zur Komposition der Trennwände 8, die aus 50% Copolymer Vinylazetat mit Äthylen und 50% Antiseptikum besteht, gehört. Nach der Füllung des Innenraums des Gehäuses 1 befestigt man an jedem Stirnende ein Aufsatzstück 2. Der Teil 9 des einen Aufsatzstücks 2 ist mit Wirkstoff 7 gefüllt. Der Teil 9 des anderen Aufsatzstücks 2 ist mit einer Komposition gefüllt, die der Komposition, die die Trennwand 8 bildet, ähnlich ist. An den Stirnenden des Gehäuses 1 werden die Netze 5 angebracht, die ein Ausschütten des Inhalts des Teils 9 sowie das Verstopfen der Öffnung 4 durch den Mageninhalt verhindern.

Bei der Vorrichtung, die mit einem Einsatz 10 (Fig. 5) versehen ist, welcher den Innenraum des Gehäuses 1 in vier Teile aufteilt, werden zunächst diejenigen Teile des Gehäuses 1 beschickt, die mit dem Funktionierungsmedium über die Öffnungen 4 des Aufsatzstücks 2 in Verbindung stehen. In diese Teile füllt man nacheinander die Abteilungen 6 mit Wirkstoff 7, die durch Trennwände 8 getrennt sind. Danach bringt man an den Stirnenden des Gehäuses 1 die Aufsatzstücke 2 an. Anschliessend füllt man diejenigen Teile des Gehäuses 1, die mit dem Funktionierungsmedium über die Öffnungen 11 (Fig. 4) in Verbindung stehen. Nach der Vorbereitung der Vorrichtung zum Einsatz bringt man diese im Magen des Wiederkäuers unter, wo sie durch Eigengewicht im Pansen oder im Netzmagen verbleibt und von wo sie nicht gerülpst werden oder in die anderen Magenteile wandern kann. Die im Magen der Wiederkäuer befindliche Flüssigkeit gelangt durch das Netz 5 (Fig. 1) zu dem Wirkstoff 7 - Tividin - im Teil 9 des Aufsatzstücks 2, löst ihn auf, und das·Tividin gelangt in Form einer Lösung in den Magen des Tieres, wobei die Ausscheidung des gesamten Tividins aus dem Teil 9 des Aufsatzstücks 2 die Ausscheidung einer erhöhten Dosis des Tividins in der Anfangsperiode der Funktion der Vorrichtung gewährleistet. Nach Ausscheidung des Tividins aus dem Teil 9 des Aufsatzstücks 2 diffundiert die Flüssigkeit aus dem Magen über die Öffnung 4 zum Wirkstoff 7 in der Trennwand 8; löst ihn auf und diffundiert in Form einer Lösung in das Funktionierungsmedium durch die Öffnung 4 wieder zurück. Der Durchmesser der Öffnung gestattet es, den Eintritt der Flüssigkeit aus dem Medium ins Innere des Gehäuses 1 und die Erweiterung der Kontaktfront des Funktionierungsmediums mit dem Wirkstoff 7 und folglich den Eintritt des Wirkstoffs 7 aus der Trennwand 8 in das Funktionierungsmedium zu kontrollieren. Bei Auflösung des Wirkstoffes 7 und dessen Auswaschung aus der Trennwand 8 bildet sich in der letzteren eine poröse Struktur. Im weiteren erfolgen ein ständiger Eintritt der Flüssigkeit zum Wirkstoff 7 in der Abteilung 6 und die Herausführung der Lösung des Wirkstoffs 7 aus dem Gehäuse 1 in das Funktionierungsmedium durch die poröse Struktur der Trennwand 8 und die Öffnung 4 des Aufsatzstücks 2. Dies gestattet es, eine Dosierung des Wirkstoffes 7 im Laufe einer längeren Zeit mit vorgegebener Konzentration durchzuführen. Nach Auflösung des gesamten Wirkstoffes 7 in der Abteilung 6 beginnt die Auflösung und Auswaschung des Wirkstoffes aus der Trennwand 8, dann aus einer anderen Abteilung 6 und so weiter, bis der gesamte Wirkstoff 7 aus dem Gehäuse 1 der Vorrichtung herausgeführt ist. Solch eine Aufeinanderfolge der Trennwände 8 und der Abteilungen 6 mit Wirkstoff 7 erlaubt es, die Dosierung des Wirkstoffes 7 im Laufe einer Zeit von einhundertachtzig bis zweihundert Tagen bei Ausscheidung der Heil- und Prophylaxe - Dosis des Tividins zu gewährleisten.

Falls die Vorrichtung einen Einsatz 10 (Fig. 2) aufweist, der den Innenraum des Gehäuses 1 in zwei isolierte Teile aufteilt, arbeitet die Vorrichtung ähnlich wie die Vorrichtung ohne Einsatz 10. Dadurch aber, dass die Dosierung der Wirkstoffe 7 aus zwei Öffnungen 4 der Aufsatzstücke 2 an zwei Stirnenden des Gehäuses 1 stattfindet, gestattet die Vorrichtung eine gleichzeitige Dosierung von mindestens zwei Wirkstoffen 7. Dabei sind die Dosiergeschwindigkeiten eines jeden der Wirkstoffe 7 verschieden. Dies ist darauf zurPuckzuführen, dass aus einem Teil des Gehäuses 1 der Wirkstoff 7, z.B. Tividin, im Laufe der gesamten Weideperiode der Tiere, bis zu zweihundert Tagen, dosiert wird. Aus dem anderen Teil des Gehäuses 1 wird der Wirkstoff 7 - das Antiseptikum - im Laufe von sechzig Tagen dosiert.

Falls die Vorrichtung einen Einsatz 10 (Fig. 3) aufweist, der den Innenraum des Gehäuses 1 in vier Teile aufteilt, arbeitet die Vorrichtung ähnlich der Vorrichtung ohne Einsatz 10. Dadurch aber, dass die Dosierung der Wirkstoffe 7 aus mehreren Punkten des Gehäuses 1 durch die Öffnungen 11 und durch die Öffnungen 4 stattfindet, gestattet die Vorrichtung eine gleichzeitige Dosierung von mehreren Wirkstoffen 7. Dank dem Vorhandensein eines zusätzlichen Volumens des Wirkstoffs 7 im Teil 9 des Aufsatzstücks 2 des Gehäuses 1 gestattet es die Vorrichtung, in der Anfangsperiode der Funktion eine höhere Dosis an Wirkstoff 7 auszu-scheiden. Durch die unterschiedliche Aufeinander-folge in einem jeden Teil des Gehäuses 1 von Abteilungen 6 mit Wirkstoff 7 und von Trennwänden 8 mit Wirkstoff 7 sowie durch einen kontrollierbaren Eintritt der Flüssigkeit aus dem Funktionierungsmedium ins Innere des Gehäuses 1 in dessen jeden Teil bzw. durch Erweiterung der Kontaktfront des Funktionierungsmediums mit dem Wirkstoff 7 in jedem Teil wird es ermöglicht, eine Dosierung von mehreren Wirkstoffen 7 im Laufe einer längeren Zeit mit einer vorgegebenen Konzentration durchzuführen. In diesem Falle gestattet es die Vorrichtung, den Wirkstoff 7 - Tividin im Laufe von zweihundert Tagen, die Glukose im Laufe von einhundert-zwanzig Tagen, das Antibiotikum im Laufe von dreissig Tagen und das Antiseptikum im Laufe von sechzig Tagen zu dosieren.

Somit gestattet es die vorliegende Erfindung, auf Grund eines kontrollierbaren Eintritts der Flüssigkeit aus dem Funktionierungsmedium ins Innere des Gehäuses 1 und der Kontaktfront des Funktionierungsmediums mit dem Wirkstoff 7 sowie dank dem Vorhandensein eines zusätzlichen Volumens an Wirkstoff 7 im Gehäuse 1, eine Dosierung des Wirkstoffs 7 im Laufe einer längeren Zeit mit vorgegebener Konzentration vorzunehmen sowie mehrere Wirkstoffe 7 gleichzeitig zu dosieren und in der Anfangsperiode des Funktionierens eine erhöhte Dosis an Wirkstoff 7 auszuscheiden.

Gewerbliche Verwertbarkeit

Die Erfindung kann verwendet werden als eine Antihelminthen-Behandlungseinrichtung zur Heilung und Prophylaxe bei Wiederkäuern, die während der Sommerweidesaison gemästet werden. Ausserdem kann die Erfindung in der Medizin, in der chemischen und in der Photoindustrie angewendet werden.

**Ansprüche**

1. Vorrichtung zur kontrollierbaren Dosierung von Wirkstoffen in ein Funktionierungsmedium, die ein zylinderförmiges Gehäuse (1) mit mindestens einem Mittel zur Verbindung des Gehäuses (1) mit dem Funktionierungsmedium und im Innern des Gehäuses hintereinander angeordnete Abteilungen, die mit Wirkstoff gefüllt und durch Trennwände getrennt sind, enthält, dadurch **gekennzeichnet**, dass zumindest an einem Stirnende des Gehäuses (1) auf der Seite der Unterbringung des Funktionierungsmediums ein Netz (5) und ein Aufsatzstück (2) angebracht ist, das in Form einer Hülse mit einem in Richtung des Funktionierungsmediums konkaven Boden ausgebildet ist, von dem mindestens ein Teil die Form eines Kegelstumpfes hat, bei dem die kleinere Grundfläche eine Öffnung (4) aufweist, die das Verbindungsmittel des Gehäuses (1) mit dem Funktionierungsmittel darstellt, jede Trennwand (8) in Form einer polymeren Matrize mit darin dispergiertem Wirkstoff (7) ausgeführt ist, wobei die Konzentration des Wirkstoffs (7) in der polymeren Matrize in den Grenzen von 1 bis 70% liegt.

2. Vorrichtung zur kontrollierbaren Dosierung von Wirkstoffen in ein Funktionierungsmedium nach Anspruch 1, dadurch **gekennzeichnet**, dass sie mit einem Einsatz (10) versehen ist, der im Innern des Gehäuses (1) untergebracht und an den Seitenwänden des Gehäuses (1) befestigt ist; wobei der Einsatz (10) den Innenraum des Gehäuses (1) in zwei voneinander isolierte Teile aufteilt, an jedem Gehäusestirnende ein Netz (5) und ein Aufsatzstück (2) angebracht ist, dessen Öffnung (4) mit dem entsprechenden Teil des Innenraums des Gehäuses (1) verbunden ist.

3. Vorrichtung zur kontrollierbaren Dosierung von Wirkstoffen in ein Funktionierungsmedium nach Anspruch 1, dadurch **gekennzeichnet,** dass sie mit einem Einsatz (10) versehen ist, der im Innern des Gehäuses (1) untergebracht und an dessen Seitenwänden befestigt ist, wobei der Einsatz (10) den Innenraum des Gehäuses (1) mindestens in drei voneinander isolierte Teile aufteilt, und an jedem Stirnende des Gehäuses (1) ein Netz (5) und ein Aufsatzstück (2) angebracht ist, dessen Öffnung (4), die ein Mittel zur Verbindung des Gehäuses (1) mit dem Funktionierungsmedium darstellt, mit dem entsprechenden Teil des Innenraums des Gehäuses (1) verbunden ist, in den anderen Teilen des Innenraums des Gehäuses (1) das Mittel zur Verbindung des Gehäuses (1) mit dem Funktionierungsmedium in Form einer Öffnung (11) in der Seitenwand des Gehäuses (1) ausgebildet ist.

FIG.1

FIG.2

FIG.3

FIG.4

FIG.5

# INTERNATIONAL SEARCH REPORT

International Application No PCT/SU 89/00108

**I. CLASSIFICATION OF SUBJECT MATTER** (if several classification symbols apply, indicate all) [4]

According to International Patent Classification (IPC) or to both National Classification and IPC

Int. Cl.[5] A61K 9/52

**II. FIELDS SEARCHED**

Minimum Documentation Searched [7]

| Classification System | Classification Symbols |
|---|---|
| | |

Int. Cl.[4]  A61K 9/22 – 9/26, 9/52, A61M 31/00

Documentation Searched other than Minimum Documentation
to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | US, A, 4612088 (Alza Corporation), 16 September 1986 (16.09.86), the abstract, the claims | 1-3 |
| | & GB 2140687 | |
| | DE 3417113 | |
| | FR 2545721 | |
| | JP 60041609 | |
| | --- | |
| A | US, A, 4608048 (Alza Corporation), 26 August 1986 (26.08.86), the abstract , the claims | 1-3 |
| | & GB 2167972 | |
| | DE 3542888 | |
| | JP 61140519 | |
| | FR 2574292 | |
| | --- | |
| | ./. | |

* Special categories of cited documents: [14]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art.
"&" document member of the same patent family

**IV. CERTIFICATION**

| Date of the Actual Completion of the International Search | Date of Mailing of this International Search Report |
|---|---|
| 23 November 1989 (23.11.89) | 8 January 1990 ( 8 .01.90) |
| International Searching Authority | Signature of Authorized Officer |
| ISA/SU | |

| III. DOCUMENTS CONSIDERED TO BE RELEVANT (CONTINUED FROM THE SECOND SHEET) | | |
|---|---|---|
| Category | Citation of Document, with indication, where appropriate, of the relevant passages | Relevant to Claim No |
| A | US, A, 3732865, (Alza Corporation), 15 May 1973 (15.05.73), the abstract , the claims | 1-3 |
| A | US, A, 4416659 (Eli Lilly and Company), 22 November 1983 (22.11.83), the abstract, the claims<br>&      EP 79724<br>      GB 2109232<br>      JP 58088310 | 1-3 |
| A | US, A, 4595583 (Alza Corporation), 17 June 1986 (17.06.86), the abstract, the claims<br>&      GB 8431661<br>      DE 3509410<br>      FR 2561103<br>      JP 60236665 | 1-3 |
| A | US, A, 4675174 (Alza Corporation), 23 June 1987 (23.06.87), the abstract, the claims<br>&      GB 8618976<br>      DE 3626415<br>      FR 2586190 | 2-3 |
| A,E | SU, A3, 1484280 (Eli Lilli and Co) 30 May 1989 (30.05.89), the abstract<br>&      EP 164241<br>      JP 61002850 | 2-3 |